# EUROPÄISCHE PATENTSCHRIFT

(11) **EP 2 314 331 B1**
(45) Veröffentlichungstag und Bekanntmachung des Hinweises auf die Patenterteilung: **11.12.2013**
(21) Anmeldenummer: 09075475.5
(22) Anmeldetag: 23.10.2009
(51) Int. Cl.: A61M 1/10, F16C 1/02

(54) **Katheterpumpenanordnung und flexible Wellenanordnung mit einer Seele**
Catheter pump arrangement and flexible shaft arrangement with a cable core
Agencement de pompes de cathéter et agencement d'arbres flexible doté d'une âme

(43) Veröffentlichungstag der Anmeldung: 27.04.2011
(73) Patentinhaber: ECP Entwicklungsgesellschaft mbH, 12247 Berlin (DE)
(72) Erfinder: Röhn, Daniel, 12437 Berlin (DE); Liebing, Reiner, 14469 Potsdam (DE)
(74) Vertreter: Pfenning, Meinig & Partner GbR

(56) Entgegenhaltungen:
- EP-A1- 0 916 359
- WO-A1-2007/057132
- DE-A1- 19 962 073
- FR-A- 1 418 339
- GB-A- 562 156
- US-A- 5 300 112
- US-A- 5 820 464
- US-A1- 2008 306 327

## Beschreibung

Die Erfindung liegt auf dem Gebiet des Maschinenbaus und befasst sich speziell mit der Übertragung von Bewegungen bzw. Drehmomenten über flexible Wellen.

Derartige flexible Wellen erlauben für bestimmte Anwendungen die Übertragung von Drehmomenten, beispielsweise an schwer zugängliche Stellen, z. B. für Heimwerkergeräte oder für zahnärztliche Anwendungen, wo Bearbeitungswerkzeuge in den Mund eines Patienten eingeführt werden müssen. Generell bieten sich kleine flexible Wellen zum Einsatz im medizinischen Bereich an, beispielsweise auch für den Antrieb von Mikropumpen, wie Herzkatheterpumpen, die in einem Körper zur Förderung von körpereigenen Flüssigkeiten eingebracht werden, oder für den Einsatz bei Mikrofräsen, die zur Entfernung von Gewebe oder Ablagerungen verwendet werden können.

Es sind verschiedene Probleme beim Betrieb solcher flexibler Wellen, insbesondere bei hohen Drehzahlen, bekannt, wobei eines dieser Probleme darin liegt, dass durch Unwuchten oder sonstige mechanische Unregelmäßigkeiten eine starke Geräuschentwicklung einerseits und auch mechanisches Schlagen mit sich daraus ergebender hoher Abnutzung entstehen kann. Zudem ist oft bei möglichst kleinem Durchmesser solcher flexibler Wellen ein möglichst hohes Drehmoment bei geringer Masse der Welle zu übertragen, was mit dem Wunsch nach möglichst engen Biegeradien oft schwierig zu vereinbaren ist.

Aus dem Stand der Technik sind zur Weiterentwicklung solcher flexibler Wellen verschiedene Vorschläge bekannt. Beispielsweise ist der deutschen Offenlegungsschrift DE 101 13 208 A1 zu entnehmen, dass bei einer Hohlwelle, die in Form einer gewickelten federnden Spule vorliegt, einzelne Windungen weggelassen werden, um die Steifigkeit der Welle über die Länge zu variieren. Dadurch soll insgesamt eine bessere Biegbarkeit erreicht werden, und zudem sollen unregelmäßige Drehungen und Schwingungen vermieden werden.

Aus der DE-OS 42 08 221 A1 ist grundsätzlich eine aus zwei gegensinnig gewickelten Federdrahtspulen bestehende Hohlwelle bekannt, die in beiden Drehrichtungen Drehmoment übertragen kann.

Aus der DE-OS 29 08 143 ist eine Welle bekannt, die aus Monofil-Strängen gewickelt ist und über ihre Länge eine gleich bleibende Steifigkeit aufweist.

Die US-Patentschrift 5 108 411 offenbart eine Welle, die bezüglich ihrer Länge in verschiedene Abschnitte aufgeteilt ist, von denen wenigstens einer eine hohlwellenartige Struktur aufweist, wobei an den hohlwellenartigen Abschnitt ein massiver Wellenabschnitt angeschlossen ist. Auf diese Weise wird erreicht, dass die Welle sich in flexible und weniger flexible Längsabschnitte aufteilt. Die flexibleren Wellenabschnitte bestehen dabei aus einer spulenartig doppellagig gegensinnig gewickelten Hohlwelle.

Aus der US 2008/0306327 ist eine flexible Welle für den rotierenden Betrieb bekannt, die zur Beeinflussung des Schwingungsverhaltens entweder aus verschiedenen, hintereinander gereihten Segmenten, aus einem strangförmigen Element mit außen aufgesetzten Verstärkungshülsen oder aus einer gewickelten Spule mit über die Länge veränderlichen mechanischen Eigenschaften besteht.

Der vorliegenden Erfindung liegt vor dem Hintergrund des Standes die Technik die Aufgabe zugrunde, mit möglichst geringen konstruktiven Mitteln eine Herzkatheterpumpenanordnung mit einer Wellenanordnung sowie eine flexible Wellenanordnung zu schaffen, die einem mechanischen Aufschwingen und Schlagen der Welle sowie auch entsprechender Geräuschentwicklung Widerstände entgegensetzt.

Die Aufgabe wird gemäß der Erfindung mit den Merkmalen des Patentanspruchs 1 gelöst. Dabei ist bezüglich einer Herzkatheterpumpenanordnung mit einer Blutpumpe sowie mit einer Wellenanordnung mit einer flexiblen, schnell rotierenden Welle mit einem antriebsseitigen Ende und einem abtriebsseitigen Ende und zwischen den Enden unveränderlichem Außendurchmesser vorgesehen, dass die Welle in wenigstens zwei verschiedenen, einstückig zusammenhängenden axialen Abschnitten durch zusätzliche Verstärkungskörper verschieden große Steifigkeit und /oder Flexibilität aufweist.

Dies kann beispielsweise derart realisiert sein, dass die Steifheit der Welle von dem Endbereich, der am abtriebsseitigen Ende an den Pumpenkopf und/oder Pumpenrotor angrenzt, zum proximalen Ende der Welle hin in wenigstens einem Schritt oder kontinuierlich abnimmt.

Unter der Steifigkeit wird dabei der Widerstand verstanden, der einer Biegung der Welle entgegengesetzt wird. Die Flexibilität umfaßt auch die Eigenschaft der Welle, überhaupt unterhalb eines bestimmten Biegeradius biegbar zu sein.

Verschiedene Steifigkeiten /Flexibilitäten können durch unterschiedliche Querschnitte der Welle, unterschiedliche materielle Beschaffenheit oder die Verstärkung durch in die Welle eingeschobene Verstärkungselemente realisiert sein. Derartige Verstärkungselemente können beispielsweise in eine Hohlwelle abschnittsweise eingeschoben sein.

Bezüglich einer flexiblen Wellenanordnung ist vorgesehen, dass die Wellenanordnung eine durchgehende flexible Welle aufweist, die zwischen einem antriebsseitigen und einem abtriebsseitigen Ende wenigstens einen Hohlraum aufweist, wobei die Welle abschnittsweise durch eine in einem Hohlraum verlaufende Seele verstärkt ist.

In den durch die Seele verstärkten Abschnitten ist die Flexibilität der Wellenanordnung geringer als in den nicht durch die Seele verstärkten Abschnitten, und entsprechend können bei einer Biegebeanspruchung in den Abschnitten mit einer Seele keine geringen Biegeradien erreicht werden. Im Ausgleich dafür ist in diesen Bereichen die Steifigkeit und Stabilität der Wellenanordnung verbessert. Dadurch, dass durch die Positionierung der Hohlwellenabschnitte, in denen eine Seele verläuft, die versteiften Abschnitte frei gewählt/ positioniert werden können, kann mit der Wellenanordnung eine Gestaltung erreicht werden, die gemäß den Anforderungen im konkreten Einsatzfall an den gewünschten Stellen einen steiferen Wellenverlauf erzielt als an anderen Stellen und in bestimmten anderen Abschnitten starke Biegungen, d. h. kleine Biegeradien, zulässt.

Es können ein oder mehrere Abschnitte entlang der Wellenanordnung vorgesehen sein, in denen jeweils eine Seele angeordnet ist, wobei die Seelen voneinander axial beabstandet sind. In den durch eine Seele verstärkten Abschnitten können jeweils gleichartige Seelenabschnitte vorgesehen sein oder auch unterschiedliche Seelenabschnitte.

Jeder der Seelenabschnitte kann entlang seiner Länge ein Steifigkeitsprofil aufweisen, beispielsweise wenigstens ein Ende, an dem die jeweilige Seele in zunehmendem Maße flexibler wird. Dadurch wird ein Abknicken der hohl ausgebildeten Teile der Welle über dem Ende einer Seele vermieden und das Steifigkeitsprofil verstetigt. Die Welle kann dabei auch komplett als Hohlwelle ausgebildet sein

Dabei kann vorteilhaft vorgesehen sein, dass die Seele in dem Bereich, über den sie sich erstreckt, mit der Welle rotiert.

Die jeweilige Seele kann passgenau in die Hohlwellenabschnitte hineinpassen, so dass auch die Biegungskraftübertragung unmittelbar stattfindet und die Seele somit schon bei großen Biegeradien Kräfte mit aufnimmt. In diesem Fall ist es sinnvoll, wenn die jeweilige Seele mit der Welle rotiert, um keine Reibungsverluste zu erzeugen.

Dabei kann die Seele bzw. der jeweilige Seelenabschnitt auch Drehmoment übertragen. Hierzu ist es sinnvoll, eine möglichst drehfeste Verbindung zwischen dem Hohlwellenabschnitt und der Seele herzustellen. Die Seele kann zu diesem Zweck in die Hohlwelle eingepresst sein.

Die einzelne Seele oder der einzelne Seelenabschnitt kann vorteilhaft aus mehreren Verseilelementen durch Verseilung hergestellt sein. Durch diese Herstellungsart lässt sich die Dicke und Biegsamkeit der Seele wunschgemäß einrichten, und die Seele wird weitgehend elastisch verformbar. Die einzelnen Verseilelemente können vorteilhaft als Drähte ausgebildet sein. Somit wird die Herstellung der Seele einfach und kostengünstig.

Die Welle kann ihrerseits als Hohlwelle in Form von wenigstens einer gewickelten Schraubenfeder ausgeführt sein. Beispielsweise kann eine derartige Schraubenfeder so eng gewickelt sein, dass die Windungen unmittelbar aneinander anliegen. Eine derartige Hohlwelle ist sehr flexibel und kann dennoch Drehmoment übertragen.

Es findet bei einer derartigen Hohlwelle auch während einer sehr schnellen Drehbewegung keine zu starke Materialumformung statt, so dass die Wärmeentwicklung sowie die bei der Rotation zu leistende Umformarbeit begrenzt bleiben. Da eine in einem Sinne gewickelte Schraubenfeder Drehmoment in einer Richtung besser überträgt als in der entgegengesetzten Drehrichtung, kann es vorteilhaft sein, dass die Hohlwelle aus zwei koaxialen, gegensinnig gewickelten, ineinander geschachtelten Schraubenfedern besteht. In diesem Fall überträgt eine der Schraubenfedern in einem ersten Drehsinn das Drehmoment optimal, während die andere Schraubenfeder im entgegengesetzten Drehsinn optimal überträgt. Dennoch ergibt sich eine große Flexibilität gegenüber Biegungen. Die beiden Schraubenfedern können derart ineinander geschachtelt sein, dass die größere Schraubenfeder die kleinere Schraubenfeder unmittelbar im Presssitz umgibt. Damit ist das mechanische Spiel sowohl in axialer Richtung als auch in Drehrichtung minimiert.

Die Seele kann vorteilhaft im Inneren des Hohlwellenabschnittes zumindest an einer Stelle an diesem befestigt sein. Damit wird zuverlässig eine Verschiebung der Seele in Axialrichtung verhindert. Die Verbindung kann beispielsweise durch Löten oder Schweißen hergestellt sein.

Erfindungsgemäß ist vorgesehen, dass die Seelenabschnitte im Inneren der Hohlwelle durch wenigstens einen axialen Abstandshalter gehalten sind. Dieser kann den Abstand des jeweiligen Seelenabschnitts zum Ende der Hohlwelle bzw. der Wellenanordnung festlegen und/oder auch den Abstand zwischen zwei Seelenabschnitten.

Der jeweilige Abstandshalter weist einen geringeren Durchmesser auf als die Seele. Dadurch ist sichergestellt, dass der/die Abstandshalter keinen Betrag zu einer ungewollten Versteifung der Wellenanordnung in den Bereichen leisten, in denen keine Verstärkung durch eine Seele vorgesehen ist.

Besonders vorteilhaft können die Abstandshalter als Faden ausgebildet sein. In diesem Fall ist es sinnvoll, dass der Faden zu beiden Enden der Wellenanordnung befestigt ist und dass einzelne Seelenabschnitte auf dem Faden in axialer Richtung festgelegt sind. Der Faden selbst überträgt kein Drehmoment, und er trägt auch nichts zur Steifigkeit der Wellenanordnung bei.

Damit lässt sich die gewünschte Steifigkeit und Flexibilität abschnittsweise bei der Wellenanordnung wunschgemäß einstellen. Durch die Positionierung einzelner Seelenabschnitte auf einem Faden lässt sich beim Einsatz der Wellenanordnung kurzfristig die Positionierung der versteiften Teile der flexiblen Wellenanordnung anpassen.

Vorteilhaft kann bei der erfindungsgemäßen Wellenanordnung vorgesehen sein, dass die Wellenanordnung ein distales, abtriebsseitiges Ende zum Anschluss eines antreibbaren Aggregats und ein proximales, antriebsseitiges Ende zum Anschluss an einen Motor aufweist und dass angrenzend an das distale Ende ein seelenloser Hohlwellenabschnitt vorgesehen ist.

Eine solche Gestaltung ist insbesondere von Vorteil, wenn das distale Ende besonders stark und mit geringem Widerstand gebogen werden soll, beispielsweise bei medizinischen Anwendungen, bei denen das eigentliche Aggregat am Kopf eines Katheters und damit am distalen Ende der Wellenanordnung sitzt und entsprechend möglichst flexibel gehandhabt werden muss. Beispielsweise kann am Ende der Wellenanordnung eine Herzpumpe angeordnet sein, die durch einen Aortenbogen hindurchgeführt werden muss.

Die proximalen Bereiche der Wellenanordnung können dann relativ steif sein, da sie durch gestreckt verlaufende Blutgefäße geführt werden. In diesen Bereichen ist eine gewisse Steifigkeit der Wellenanordnung wichtiger als die Flexibilität.

Es kann jedoch auch vorteilhaft vorgesehen sein, den distalen (abtriebsseitigen) Endbereich, insbesondere den unmittelbar an ein angeschlossenes anzutreibendes Aggregat angrenzenden Bereich, beispielsweise mindestens die letzten 1%, 5% oder 10% der Länge der Welle steifer auszubilden als den Durchschnitt der gesamten Länge der Welle oder wenigstens von diesem Bereich ausgehend die Steifheit der Welle kontinuierlich oder sprunghaft zum proximalen Ende hin zu verringern.

Damit wird ein guter Rundlauf im Endbereich erzielt.

In diesem Fall weist die Wellenanordnung einen die Hohlwelle umgebenden Hohlkatheter auf, um die Wellenanordnung insgesamt zu schützen und gegenüber äußeren Einflüssen abzuschirmen. Im Inneren des Hohlkatheters kann beispielsweise ein Kühl- und Schmiermittel für die Welle vorgesehen sein.

Eine besonders vorteilhafte medizinische Anwendung der Erfindung sieht die Ausstattung einer Herzkatheterpumpenanordnung mit einer flexiblen Wellenanordnung gemäß der obigen Beschreibung vor.

Im Folgenden wird die Erfindung anhand eines Ausführungsbeispiels in einer Zeichnung gezeigt und nachfolgend beschrieben.

Dabei zeigt
- Fig. 1: eine erfindungsgemäße Wellenanordnung in einer dreidimensionalen Darstellung mit mehreren durch eine Seele verstärkten Ab- schnitten,
- Fig. 2: einen Querschnitt der Wellenanordnung aus Fig. 1,
- Fig. 3: eine weitere Ausführungsform der Wellenan- ordnung in einer schematischen Darstellung,
- Fig. 4: eine Ausführung der erfindungsgemäßen Wellenanordnung in gebogener Form und
- Fig. 5: die Anwendung der erfindungsgemäßen Wellen- anordnung bei einer Herzpumpe.

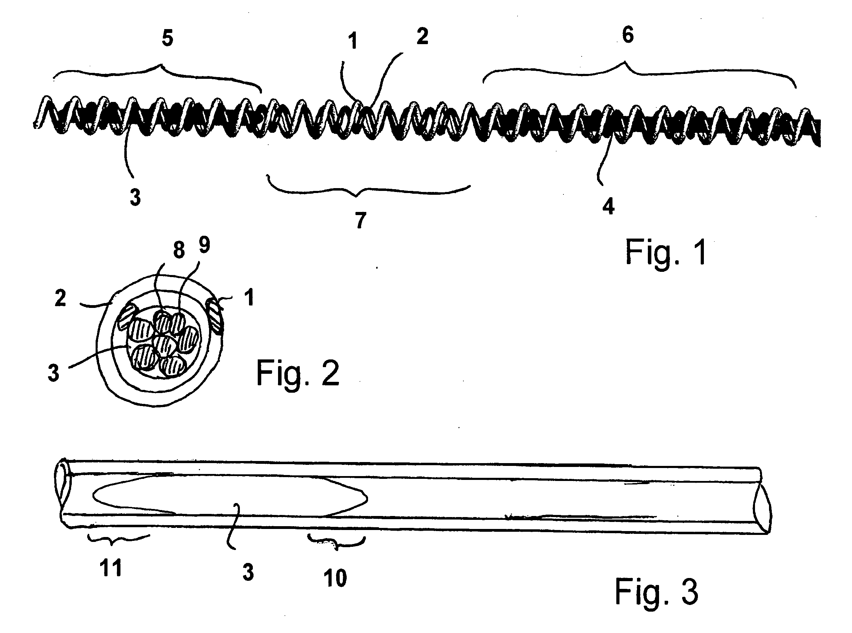

Die Fig. 1 zeigt in dreidimensionaler Ansicht eine Hohlwelle, die aus zwei gegensinnig gewickelten Schraubenfedern 1, 2 besteht, von denen die erste hell, die zweite dunkel dargestellt ist. Die gegensinnige Wicklung der beiden Schraubenfedern bewirkt, dass in jeder Drehrichtung eine der Federn gestaucht und die andere gestreckt wird. Somit findet insgesamt keine Verformung in Axialrichtung abhängig von der Drehrichtung statt, die zu übertragen ist.

Die Dichte der Wicklungen der einzelnen Federn sowie die Stärke des Federdrahtes bestimmen einerseits die Flexibilität bzw. Steifigkeit der Hohlwelle, andererseits das übertragbare Drehmoment.

Es sind weiterhin in der Fig. 1 zwei Seelenabschnitte 3, 4 dargestellt, die die Wellenanordnung jeweils in den Axialabschnitten 5, 6 versteifen. In dem dazwischen liegenden Axialabschnitt 7 bleibt die Hohlwelle frei und ist dort entsprechend flexibler.

Die Seelenabschnitte 3, 4 können als massive Körper, beispielsweise als Kunststoff- oder Metallkörper ausgebildet sein, die eine hohe Federelastizität und Bruchfestigkeit sowie einen hohen Ermüdungswiderstand aufweisen.

Die Seelenabschnitte können jedoch auch als verseilte Seelen ausgeführt sein, die dann aus einer Vielzahl von Verseilelementen, beispielsweise Drähten, bestehen. Diese Ausführungsform ist näher anhand der Fig. 2 dargestellt, wo ein Schnitt die radiale Anordnung der beiden Schraubenfedern 1, 2 sowie der Seele 3 zeigt. Es ist dort auch dargestellt, dass die Seele 3 aus einer Vielzahl von Verseilelementen 8, 9 besteht, wodurch sie sehr flexibel und dauerhaft verformbar wird.

Die beiden Schraubenfedern 1, 2 sind derart bemessen und angeordnet, dass sie radial ineinander koaxial zueinander im Presssitz zusammengefügt sind, so dass zwischen ihnen eine Verteilung des zu übertragenden Drehmoments stattfindet. Zudem werden auch Biegebeanspruchungen durch beide Schraubenfedern gemeinsam aufgenommen. Die entsprechenden Belastungen werden in den Abschnitten, in denen sich eine Seele innerhalb des Hohlraums der Schraubenfedern 1, 2 befindet, durch diese Seele ebenfalls mit aufgenommen, da sie eng in den Hohlraum eingepasst ist.

Anhand der Fig. 3 ist dargestellt, dass die Seelenabschnitte jeweils ein sich verjüngend zulaufendes Ende aufweisen, wodurch der Endbereich 10, 11 der Seele 3 zum Ende hin immer flexibler wird. Dadurch wird in der Gesamtbetrachtung der Wellenanordnung zum Ende eines jeden Seelenabschnitts die Steifigkeit nicht abrupt auf das Maß der Steifigkeit der Hohlwelle herabgesetzt, sondern es findet ein stetiger Übergang statt, der bei einer Biegebeanspruchung der Wellenanordnung zu einer kontinuierlichen Verteilung führt, um Knickbeanspruchungen und die Gefahr des Reißens der Wellenanordnung zu verringern.

Im Ergebnis ist bei einer gegebenen Biegebeanspruchung der Biegeradius in den Abschnitten 5, 6, in denen die Hohlwelle durch eine Seele bzw. einen Seelenabschnitt verstärkt ist, deutlich vergrößert. In den Abschnitten, in denen sich keine Seele befindet, wird ein erheblich geringerer Biegeradius erreicht. Um Knicke zwischen diesen Bereichen zu vermeiden, ist die oben beschriebene Gestaltung des Endbereichs der Seelenabschnitte geeignet.

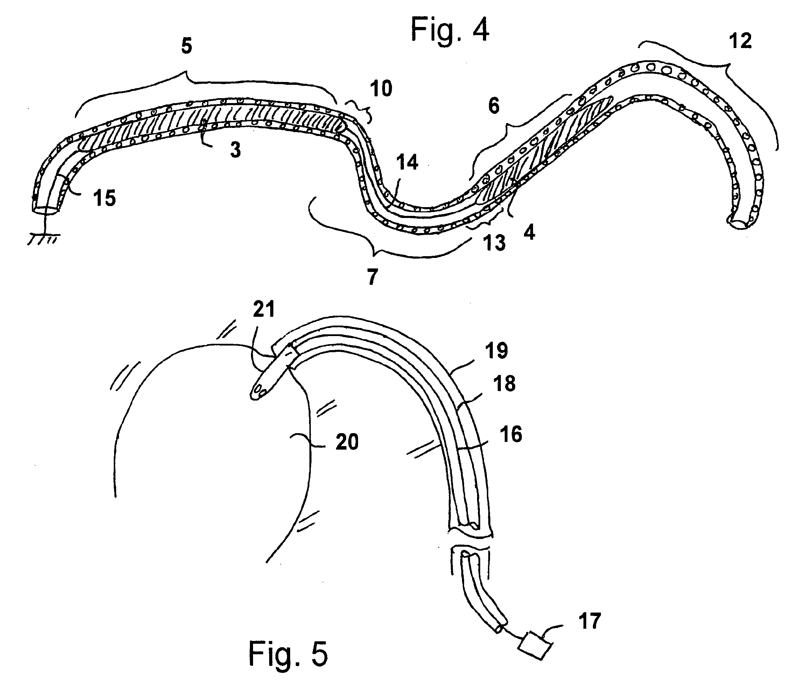

In der Fig. 4 ist beispielhaft eine gebogene Wellenanordnung gemäß der Erfindung dargestellt, wobei zwei Seelen 3, 4 dargestellt sind in Abschnitten 5, 6, die nahezu gerade verlaufen bzw. einen großen Biegeradius aufweisen. Besonders in dem Abschnitt 7 ist die Hohlwellenanordnung stark gebogen, ebenso wie im Abschnitt 12.

Der allmähliche Übergang der Steifigkeit durch entsprechende Gestaltung der Enden der Seelen 3, 4 in den Bereichen 10, 13 bewirkt, dass dort die Knickgefahr reduziert ist.

Zwischen den Seelen 3, 4 sind in der Fig. 4 beispielhaft Abstandshalter dargestellt, ebenso wie zwischen der Seele 3 und dem antriebsseitigen Ende der Wellenanordnung. Die Abstandshalter sind mit 14, 15 bezeichnet und können als mehr oder weniger steife, dünne Seelen ausgeführt sein, die einen wesentlichen geringeren Durchmesser aufweisen als die Seelen 3, 4 und ebenso eine wesentlich geringere Steifigkeit. Die Abstandshalter 14, 15 können jedoch einfach nur als Faden mit vernachlässigbarer Steifigkeit ausgebildet sein, wobei sich in diesem Fall anbietet, die Abstandshalter zu beiden Enden der Hohlwellenanordnung geeignet zu befestigen, um die Seelenabschnitte 3, 4 wie auf einer Kette gespannt und in vorgegebenen Abständen stabil halten zu können.

Diese Ausführungsform hat den Vorteil, dass in die bestehende Hohlwelle je nach den Anforderungen an die Verteilung unterschiedlicher Steifigkeiten entlang der Hohlwellenanordnung eine Reihe von Seelen/Seelenabschnitten mit Abstandshaltern eingezogen werden kann, wobei die Länge der einzelnen Abstandshalter nach dem Einsatzzweck der Wellenanordnung individuell anpassbar ist.

Die Fig. 5 zeigt schematisch einen Anwendungsfall für die erfindungsgemäße Wellenanordnung, wobei die Wellenanordnung dort nur schematisch dargestellt und mit 16 bezeichnet ist. Die Wellenanordnung 16 ist antriebsseitig mit einem Motor 17 verbunden und verläuft in einem Hohlkatheter 18, der in ein Blutgefäß 19 eines Körpers, beispielsweise eines menschlichen Körpers einführbar und über den Weg dieses Blutgefäßes in eine Herzkammer 20 einbringbar ist.

Am Ende des Hohlkatheters 18 befindet sich eine Herzpumpe 21, die als Axialpumpe ausgeführt ist und in ihrem Inneren einen Rotor aufweist, der mittels der Wellenanordnung 16 mit hohen Drehzahlen, beispielsweise zwischen 10.000 und 20.000 Umdrehungen pro Minute, antreibbar ist.

Die Vorteile der erfindungsgemäßen Wellenanordnung zeigen sich darin, dass einerseits die Wellenanordnung aufgrund geeigneter steifer Bereiche durch das Blutgefäß 19 gut einschiebbar ist, dass jedoch im distalen Bereich, von der Einbringstelle aus gesehen, also im Bereich des Aortenbogens zur Herzkammer hin, eine hohe Flexibilität der Wellenanordnung gegeben ist, so dass dort die Herzpumpe 21 in die Herzkammer einbringbar ist, ohne dass die Steifigkeit der Wellenanordnung bzw. des Hohlkatheters zu Verletzungen der Herzkammerwände oder der Aorta im Bereich des Aortenbogens führen kann. Durch die geeignete Verteilung der Seele(n) entlang der Wellenanordnung wird ein Schlagen der Welle und akustische Resonanz zuverlässig vermieden.

## Patentansprüche

1. Herzkatheterpumpenanordnung mit einer flexiblen Wellenanordnung mit einer durchgehenden flexiblen Welle (11,12) mit einem antriebsseitigen Ende und einem abtriebsseitigen Ende, wobei die Welle zwischen dem antriebsseitigen Ende und dem abtriebsseitigen Ende wenigstens einen Hohlraum aufweist und abschnittsweise durch wenigstens einen in einem Hohlraum verlaufenden Verstärkungskörper in Form einer Seele (3,4) verstärkt ist, wobei wenigstens eine Seele im Inneren der Welle durch wenigstens.einen axialen Abstandshalter gehalten ist,
**dadurch gekennzeichnet, dass** der/die Abstandshalter (14,15) einen geringeren Durchmesser aufweist/aufweisen als die verschiedenen Seelen.

2. Herzkatheterpumpenanordnung nach Anspruch 1, **dadurch gekennzeichnet, dass** die Seele (3,4) in dem Bereich, über den sie sich erstreckt, mit der Welle (1,2) rotiert.

3. Herzkatheterpumpenanordnung nach Anspruch 2, **dadurch gekennzeichnet, dass** die Seele (3,4) in dem Bereich, über den sie sich erstreckt, Drehmoment überträgt.

4. Herzkatheterpumpenanordnung nach Anspruch 1 oder einem der folgenden, **dadurch gekennzeichnet, dass** die Seele (3,4) aus mehreren Verseilelementen (8,9) durch Verseilung hergestellt ist.

5. Herzkatheterpumpenanordnung nach Anspruch 4, **dadurch gekennzeichnet, dass** die Verseilelemente (8,9) durch Drähte gebildet sind.

6. Herzkatheterpumpenanordnung nach Anspruch 1 oder einem der folgenden, **dadurch gekennzeichnet, dass** die Welle (1,2) wenigstens abschnittsweise als wenigstens eine gewickelte Schraubenfeder ausgebildet ist.

7. Herzkatheterpumpenanordnung nach Anspruch 6, **dadurch gekennzeichnet, dass** die Welle (1,2) wenigstens zwei koaxiale, gegensinnig gewickelte, ineinander geschachtelte Schraubenfedern aufweist.

8. Herzkatheterpumpenanordnung nach Anspruch 1 oder einem der folgenden, **dadurch gekennzeichnet, dass** die Welle (1,2) in wenigstens zwei voneinander beabstandeten axialen Bereichen (5,6) von einer Seele (3,4) durchsetzt ist.

9. Herzkatheterpumpenanordnung nach Anspruch 1 oder einem der folgenden, **dadurch gekennzeichnet, dass** wenigstens ein Abstandshalter (14,15) als Faden ausgebildet ist.

10. Herzkatheterpumpenanordnung nach Anspruch 1 oder einem der folgenden, **dadurch gekennzeichnet, dass** die Wellenanordnung ein distales, abtriebsseitiges Ende zum Anschluss eines antreibbaren Aggregats (21) und ein proximales, antriebsseitiges Ende zum Anschluss an einen Motor (17) aufweist und dass angrenzend an das distale Ende ein seelenloser Abschnitt der Welle (1,2) vorgesehen ist.

11. Herzkatheterpumpenanordnung nach einem der Ansprüche 1 bis 10, **dadurch gekennzeichnet dass** ein distaler (abtriebsseitiger) Endbereich, insbesondere der unmittelbar an ein angeschlossenes anzutreibendes Aggregat angrenzende Bereich, insbesondere mindestens die letzten 1%, 5% oder 10% der Länge der Welle steifer ausgebildet sind als der Durchschnitt der gesamten Länge der Welle, insbesondere steifer als alle übrigen Bereiche der Welle.

12. Herzkatheterpumpenanordnung nach Anspruch 1 oder einem der folgenden, **gekennzeichnet durch** einen die Welle (1,2) umgebenden Hohlkatheter (19) für medizinische Anwendungen.

13. Herzkatheterpumpenanordnung nach Anspruch 1 oder einem der folgenden, **dadurch gekennzeichnet, dass** die Steifheit der Welle von dem Endbereich, der an den Pumpenkopf und/oder Pumpenrotor angrenzt, zum proximalen Ende der Welle hin in wenigstens einem Schritt oder kontinuierlich abnimmt.

## Claims

1. Cardiac catheter pump arrangement with a flexible shaft arrangement having a throughgoing flexible shaft (11, 12) with an end at the drive side and an end at the output side, wherein the shaft has at least one hollow space between the end at the drive side and the end at the output side and is reinforced sectionally by at least one reinforcement body in the form of a core (3, 4) extending in a hollow space, wherein at least one core is held in the interior of the shaft by at least one axial spacer,
**characterized in that** the spacer/spacers (14, 15) has/have a smaller diameter than the different cores.

2. Cardiac catheter pump arrangement according to claim 1, **characterized in that** the core (3, 4) rotates with the shaft (1, 2) in the region over which it extends.

3. Cardiac catheter pump arrangement according to claim 2, **characterized in that** the core (3, 4) transfers torque in the region over which it extends.

4. Cardiac catheter pump arrangement according to claim 1 or one of the following claims, **characterized in that** the core (3, 4) is manufactured by stranding from a plurality of strand elements (8, 9).

5. Cardiac catheter pump arrangement according to claim 4, **characterized in that** the strand elements (8, 9) are formed by wires.

6. Cardiac catheter pump arrangement according to claim 1 or one of the following claims, **characterized in that** the shaft (1, 2) is made at least section-wise as at least one wound helical spring.

7. Cardiac catheter pump arrangement according to claim 6, **characterized in that** the shaft (1, 2) has at least two coaxial screws wound in opposite senses and nested in one another.

8. Cardiac catheter pump arrangement according to claim 1 or one of the following claims, **characterized in that** the shaft (1, 2) is penetrated by a core (3, 4) in at least two axial regions (5, 6) spaced apart from one another.

9. Cardiac catheter pump arrangement according to claims 1 or one of the following claims, **characterized in that** at least one spacer (14, 15) is made as a thread.

10. Cardiac catheter pump arrangement according to claim 1 or one of the following claims, **characterized in that** the shaft arrangement has a distal end at the output side for connection of a drivable unit (21) and a proximal end at the drive side for the connection to a motor (17) and **in that** a coreless section of the shaft (1, 2) is provided adjacent to the distal end.

11. Cardiac catheter pump arrangement according to one of claims 1 to 10, **characterized in that** a distal end region (at the output side), in particular the region directly adjacent to a connected unit to be driven, in particular at least the last 1 %, 5 % or 10 % of the length of the shaft, are made stiffer than the average of the total length of the shaft, in particular stiffer than all the other regions of the shaft.

12. Cardiac catheter pump arrangement according to claim 1 or one of the following claims, **characterized by** a a hollow catheter (19) for medical applications surrounding the shaft (1, 2).

13. Cardiac catheter pump arrangement according to claim 1 or one of the following claims, **characterized in that** the stiffness of the shaft reduces in at least one step or continuously from the end region which is adjacent to the pump head and/or to the pump rotor toward the proximal end of the shaft.

## Revendications

1. Système de pompe pour cathéter cardiaque avec un ensemble d'arbre avec un arbre souple traversant (11, 12) avec une extrémité côté entraînement et une extrémité côté sortie, dans lequel l'arbre comporte au moins un espace creux entre l'extrémité côté entraînement et l'extrémité côté sortie, tout en étant renforcé par sections par au moins un corps de renforcement s'étendant dans l'espace creux, sous la forme d'une âme (3, 4), dans lequel au moins une âme est maintenue à l'intérieur de l'arbre par au moins une entretoise axiale,
**caractérisé en ce que** l'entretoise/les entretoises (14, 15) présente/présentent un diamètre inférieur à celui des différentes âmes.

2. Système de pompe pour cathéter cardiaque selon la revendication 1, **caractérisé en ce que** l'âme (3, 4) tourne avec l'arbre (1, 2) dans la région sur laquelle elle s'étend.

3. Système de pompe pour cathéter cardiaque selon la revendication 2, **caractérisé en ce que** l'âme (3, 4) transmet un couple dans la région sur laquelle elle s'étend.

4. Système de pompe pour cathéter cardiaque selon la revendication 1 ou l'une des suivantes, **caractérisé en ce que** l'âme (3, 4) est réalisée par câblage à partir de plusieurs éléments de câblage (8, 9).

5. Système de pompe pour cathéter cardiaque selon la revendication 4, **caractérisé en ce que** les éléments de câblage (8, 9) sont formés par des fils métalliques.

6. Système de pompe pour cathéter cardiaque selon la revendication 1 ou l'une des suivantes, **caractérisé en ce que** l'arbre (1, 2) est formée au moins par sections comme au moins un ressort hélicoïdal.

7. Système de pompe pour cathéter cardiaque selon la revendication 6, **caractérisé en ce que** l'arbre (1, 2) comporte au moins deux ressorts hélicoïdaux coaxiaux engagés l'un dans l'autre et enroulés en sens inverse.

8. Système de pompe pour cathéter cardiaque selon la revendication 1 ou l'une des suivantes, **caractérisé en ce que** l'arbre (1, 2) est traversé par une âme (3, 4) dans au moins deux régions axiales (5, 6) espacées l'une de l'autre.

9. Système de pompe pour cathéter cardiaque selon la revendication 1 ou l'une des suivantes, **caractérisé en ce qu'**au moins une entretoise (14, 15) est conçue comme un fil.

10. Système de pompe pour cathéter cardiaque selon la revendication 1 ou l'une des suivantes, **caractérisé en ce que** l'ensemble d'arbre comporte une extrémité distale côté sortie, pour le raccordement d'un agrégat (21) entraînable, ainsi qu'une extrémité proximale côté entraînement, pour le raccordement à un moteur (17), et **en ce qu'**à côté de l'extrémité distale, il est prévu une section d'arbre (1, 2) sans âme.

11. Système de pompe pour cathéter cardiaque selon l'une des revendications 1 à 10, **caractérisé en ce qu'**une région d'extrémité distale (côté sortie), en particulier la région immédiatement adjacente à un agrégat raccordé à entraîner, en particulier au moins les derniers 1%, 5% ou 10% de la longueur de l'arbre, sont conçus plus rigides que la moyenne de la longueur totale de l'arbre, en particulier plus rigides que toutes les autres régions de l'arbre.

12. Système de pompe pour cathéter cardiaque selon la revendication 1 ou l'une des suivantes, **caractérisé par** un cathéter creux (19) entourant l'arbre (1, 2) pour des applications médicales.

13. Système de pompe pour cathéter cardiaque selon la revendication 1 ou l'une des suivantes, **caractérisé en ce que** la rigidité de l'arbre diminue progressivement ou soudainement en au moins un point, entre la région d'extrémité adjacente à la tête de pompe et/ou au rotor de pompe et l'extrémité proximale de l'arbre.
